(19) Europäisches Patentamt
European Patent Office
Office européen des brevets

(11) **EP 4 682 371 A2**

(12) **DEMANDE DE BREVET EUROPEEN**

(43) Date de publication:
**21.01.2026   Bulletin 2026/04**

(51) Classification Internationale des Brevets (IPC):
***F02K 9/08*** *(2006.01)*    ***F02K 9/96*** *(2006.01)*
***G01N 33/22*** *(2006.01)*

(21) Numéro de dépôt: **25188883.0**

(22) Date de dépôt: **10.07.2025**

(52) Classification Coopérative des Brevets (CPC):
**F02K 9/08; F02K 9/96; G01N 33/222; G06N 20/20**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité:   **17.07.2024   FR 2407789**

(71) Demandeur: **ARIANEGROUP SAS**
**78130 Les Mureaux (FR)**

(72) Inventeurs:
• **GALLIER, Stany**
**91710 VERT LE PETIT (FR)**
• **JAOUI, Ruben**
**91710 VERT LE PETIT (FR)**

(74) Mandataire: **Cabinet Le Guen Maillet**
**3, impasse de la Vigie**
**CS 71840**
**35418 Saint-Malo Cedex (FR)**

(54) **PROCEDE D ESTIMATION DE VITESSE DE COMBUSTION D'UNE COMPOSITION DE PROPERGOL SOLIDE**

(57)    Pour obtenir une estimation de vitesse de combustion d'une composition de propergol solide à évaluer, un système de traitement : calcule (106) une distance de similarité avec un contenu de base de données ayant servi à entraîner et valider un modèle d'apprentissage automatique pour estimer des vitesses de combustion à partir de jeux de caractéristiques représentatifs de compositions de propergol solide ; fournit (110) l'estimation de vitesse de combustion de la composition de propergol solide, en utilisant le modèle d'apprentissage automatique, lorsque la distance de similarité calculée est inférieure à un seuil prédéterminé ; et fournit (112) l'estimation de vitesse de combustion de la composition de propergol solide, en utilisant au contraire un modèle physique représentatif de lois de combustion de propergol solide, lorsque la distance de similarité calculée est supérieure ou égale au seuil prédéterminé.

Fig. 1

## Description

DOMAINE TECHNIQUE

La présente invention concerne le domaine de la prédiction de vitesse de combustion (« burning rate » en anglais) d'une composition de propergol solide.

ETAT DE LA TECHNIQUE ANTERIEURE

Les propergols solides sont des matériaux énergétiques largement utilisés pour la propulsion des fusées. On choisit généralement des propergols composites, dans lesquels des particules oxydantes sont incorporées dans un liant polymère. Par exemple, les particules oxydantes sont du perchlorate d'ammonium (AP pour « ammonium perchlorate » en anglais) et le liant polymère est du polybutadiène hydroxytéléchélique (HTPB pour « hydroxyl-terminated polybutadiene » en anglais). Classiquement, de la poudre d'aluminium (AI) est également incorporée, ce qui donne ainsi par exemple des propergols composites de type AP/HTPB/AI.

La vitesse de combustion d'un propergol solide (c'est-à-dire la vélocité de combustion à une pression donnée) est un paramètre clef pour la conception des fusées et doit être soigneusement définie afin de respecter des spécifications balistiques. Habituellement, la taille et le taux des particules oxydantes sont ajustés pour adapter la vitesse de combustion du propergol solide dans les limites de valeurs cibles spécifiées. Des catalyseurs balistiques (« burning rate modifier » en anglais) peuvent aussi être utilisés en complément, à très faible taux, pour augmenter la vitesse de combustion du propergol solide.

Pour ce faire, une phase d'expérimentations est réalisée avec différentes compositions de propergols solides. Ces expérimentations sont généralement menées en se basant principalement sur l'expérience de chimistes spécialistes des propergols solides, qui peut être toutefois limitée pour les propergols solides inhabituels ou nouveaux, par exemple dans le cas d'un développement d'une nouvelle génération de fusées. Un grand nombre de propergols solides doivent ainsi être expérimentés avant de trouver une ou plusieurs compositions qui répondent aux valeurs cibles spécifiées.

Il existe donc un besoin d'accélérer cette phase d'expérimentations en fournissant une solution permettant d'estimer de manière efficace quelle vitesse de combustion peut être attendue pour une composition donnée. Avec une telle solution, le nombre d'expérimentations peut être limité en les concentrant sur des compositions de propergol solide dont la vitesse de combustion estimée par la solution proposée correspond aux valeurs cibles spécifiées.

EXPOSE DE L'INVENTION

A cet effet, il est proposé ici un procédé pour obtenir une estimation de vitesse de combustion d'une composition de propergol solide à évaluer, le procédé étant exécuté par un système de traitement, le procédé comportant :

- calculer une distance de similarité avec un contenu de base de données ayant servi à entraîner et valider un modèle d'apprentissage automatique pour estimer des vitesses de combustion à partir de jeux de caractéristiques représentatifs de compositions de propergol solide ;
- fournir l'estimation de vitesse de combustion de la composition de propergol solide, en utilisant le modèle d'apprentissage automatique, lorsque la distance de similarité calculée est inférieure à un seuil prédéterminé ; et
- fournir l'estimation de vitesse de combustion de la composition de propergol solide, en utilisant au contraire un modèle physique représentatif de lois de combustion de propergol solide, lorsque la distance de similarité calculée est supérieure ou égale au seuil prédéterminé.

Ainsi, des phases d'expérimentations pour trouver une composition de propergol solide qui réponde à des spécifications de vitesse de combustion sont accélérées en tirant astucieusement bénéfice du modèle d'apprentissage automatique lorsqu'une composition de propergol solide à évaluer est similaire à des données qui ont servi à son apprentissage et à sa validation et de s'appuyer sur le modèle physique dans le cas contraire.

Selon un mode de réalisation particulier, la distance de similarité, notée $d_{min}$, est calculée de la façon suivante :

$$d_{min} = \min_i \sum_k w_k \cdot (f_k^i - g_k)^2$$

où $f_k^i$ est la valeur de la $k$-ième caractéristique d'un vecteur $f^i$ correspondant à un $i$-ème jeu de caractéristiques dans la base de données, $g_k$ est la valeur de la $k$-ième caractéristique d'un jeu de caractéristiques représentatif de la composition de propergol solide à évaluer $g$ et $w_k$ est un coefficient de pondération associé à la $k$-ième caractéristique.

Ainsi, la distance de similarité est aisément calculée.

Selon un mode de réalisation particulier, les coefficients de pondération $w_k$, $k=1,..,N$, sont définis par une analyse de la base de données par un modèle d'apprentissage automatique de type forêt d'arbres décisionnels.

Ainsi, il est efficacement tenu compte de l'influence de chaque caractéristique dans l'établissement de la vitesse de combustion.

Selon un mode de réalisation particulier, le modèle d'apprentissage automatique pour estimer des vitesses de combustion à partir de compositions de propergol solide est de type réseau de neurones artificiels.

Selon un mode de réalisation particulier en variante, le modèle d'apprentissage automatique pour estimer des vitesses de combustion à partir de compositions de propergol solide est de type forêt d'arbres décisionnels extrêmes.

Ainsi, dans les deux variantes ci-dessus, de bons résultats d'estimation de vitesse de combustion sont obtenus dans l'espace des données qui ont servi à l'apprentissage et à la validation du modèle d'apprentissage automatique.

Selon un mode de réalisation particulier, la base de données contient un ensemble de jeux de caractéristiques avec lesquels le modèle d'apprentissage automatique pour estimer des vitesses de combustion à partir de compositions de propergol solide a été entraîné et validé, chaque jeu de caractéristiques étant représentatif d'une composition de propergol solide associée à une valeur de pression pour une vitesse de combustion correspondante, chaque jeu de caractéristiques comportant :

- la valeur de pression ;
- une indication de fraction massique de particules oxydantes pour chaque taille d'un panel de tailles possibles pour les particules oxydantes ;
- une indication de fraction massique d'aluminium potentiellement incorporé ;
- une indication de taille nominale de particules d'aluminium potentiellement incorporé ;
- une indication de nature d'un catalyseur balistique potentiellement incorporé ; et
- une indication de taille de particules du catalyseur balistique potentiellement incorporé.

Ainsi, le modèle d'apprentissage automatique fait preuve d'une grande précision.

Selon un mode de réalisation particulier, chaque jeu de caractéristiques comporte en outre une indication de type de malaxeur utilisé.

Ainsi, la précision du modèle d'apprentissage automatique est améliorée.

Selon un mode de réalisation particulier, l'indication de type de malaxeur utilisé est une indication de catégorie de taille de malaxeur utilisé.

Ainsi, l'amélioration de la précision du modèle d'apprentissage automatique est aisément obtenue bien que différents modèles de malaxeurs soient utilisés.

Selon un mode de réalisation particulier, une première estimation de vitesse de combustion de la composition de propergol solide est fournie en utilisant le modèle d'apprentissage automatique et une deuxième estimation de vitesse de combustion de la composition de propergol solide est fournie en utilisant le modèle physique, et dans lequel ladite première estimation est mise en avant comme estimation privilégiée lorsque la distance de similarité calculée est inférieure au seuil prédéterminé et ladite deuxième estimation est mise en avant comme estimation privilégiée lorsque la distance de similarité calculée est supérieure ou égale au seuil prédéterminé.

Ainsi, il est aisé d'ajuster éventuellement ledit seuil prédéterminé en vérifiant, à l'expérimentation, si la vitesse de combustion mesurée est plus proche de la première estimation avec le modèle d'apprentissage automatique ou de la deuxième estimation avec le modèle physique.

Il est également proposé ici un produit programme d'ordinateur comportant des instructions pour implémenter le procédé ci-dessus dans l'un quelconque de ses modes de réalisation, lorsque les instructions sont exécutées par un processeur. Il est aussi proposé ici un support de stockage d'informations stockant des instructions pour implémenter le procédé ci-dessus dans l'un quelconque de ses modes de réalisation, lorsque les instructions sont exécutées par le processeur.

Il est également proposé ici un procédé d'expérimentation pour rechercher une composition de propergol solide qui respecte une vitesse de combustion spécifiée, comportant :

- obtenir un descriptif d'une composition de propergol solide à évaluer ;
- obtenir une estimation de vitesse de combustion de la composition de propergol solide à évaluer en appliquant le procédé ci-dessus dans l'un quelconque de ses modes de réalisation ;
- lorsque l'estimation de vitesse de combustion de la composition de propergol solide à évaluer respecte une vitesse de combustion spécifiée avec une marge prédéterminée, expérimenter la composition de propergol solide en question, sinon rechercher une autre composition de propergol solide à évaluer, et réitérer.

Il est aussi proposé ici un système de traitement comportant de la circuiterie électronique configurée pour obtenir une estimation de vitesse de combustion d'une composition de propergol solide à évaluer, en :

- calculant une distance de similarité avec un contenu de base de données ayant servi à entraîner et valider un modèle d'apprentissage automatique pour estimer des vitesses de combustion à partir de compositions de propergol solide ;
- fournissant l'estimation de vitesse de combustion de la composition de propergol solide, obtenue en utilisant le modèle d'apprentissage automatique, lorsque la distance de similarité calculée est inférieure à un seuil prédéterminé ; et
- fournissant l'estimation de vitesse de combustion de la composition de propergol solide, obtenue en utilisant un modèle physique représentatif de lois de combustion de propergol solide, lorsque la distance de similarité calculée est supérieure ou égale à un seuil prédéterminé.

BREVE DESCRIPTION DES DESSINS

Les caractéristiques de l'invention mentionnées ci-dessus, ainsi que d'autres, apparaîtront plus clairement à la lecture de la description suivante d'au moins un exemple de réalisation, ladite description étant faite en relation avec les dessins joints, parmi lesquels :

[Fig. 1] illustre schématiquement un procédé d'estimation de vitesse de combustion d'une composition de propergol solide ;
[Fig. 2] illustre schématiquement un système comportant de la circuiterie électrique adaptée pour implémenter le procédé de la Fig. 1 ;
[Fig. 3] illustre schématiquement un algorithme de mise en place d'une base de données et d'un modèle d'apprentissage automatique, qui sont utilisés dans le procédé de la Fig. 1 ; et
[Fig. 4] illustre schématiquement un procédé de conduite d'expérimentations qui utilise le procédé de la Fig. 1.

EXPOSE DETAILLE DE MODES DE REALISATION

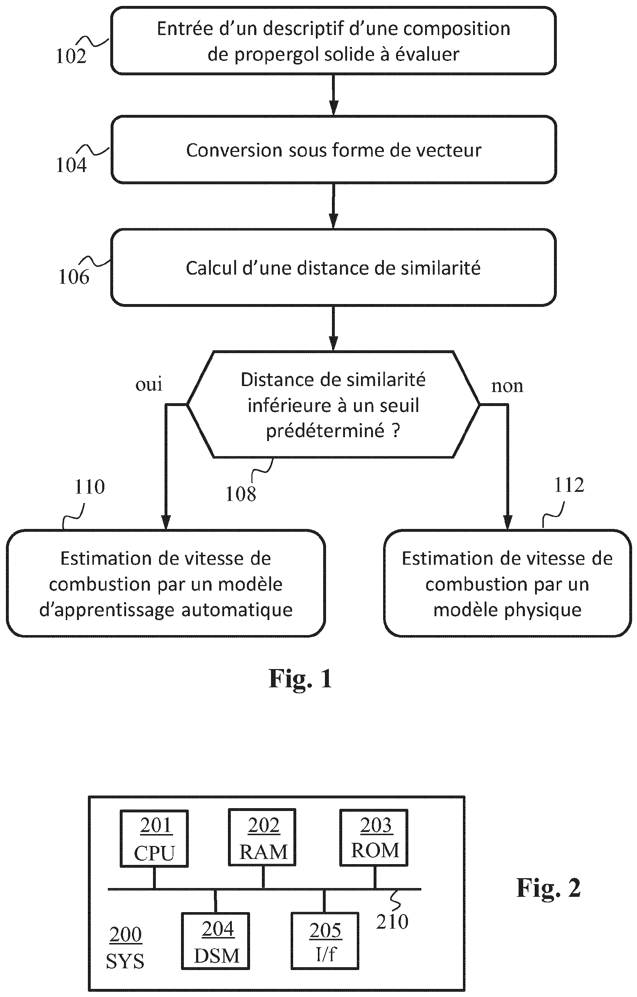

La **Fig. 1** illustre ainsi schématiquement un procédé d'estimation de vitesse de combustion d'une composition de propergol solide.
Dans une étape 102, un descriptif de composition de propergol solide à évaluer est entré dans un système de traitement (« processing system » en anglais). Par exemple, le système de traitement est un système informatique exécutant un outil logiciel fournissant une interface graphique permettant de saisir, au moins, des caractéristiques de composition de propergol solide.
Dans un mode de réalisation particulier, la composition de propergol solide en question doit être évaluée à une valeur de pression donnée.
Dans une étape 104, le système de traitement convertit préférentiellement la composition de propergol solide à évaluer sous forme de vecteur, de manière à en permettre un traitement numérique aisé.
Dans une étape 106, le système de traitement effectue un calcul de distance de similarité avec un contenu de base de données ayant servi à entraîner et valider un modèle d'apprentissage automatique, noté ici MLMOD, pour estimer des vitesses de combustion à partir de compositions de propergol solide. Le calcul de distance de similarité permet d'évaluer dans quelle mesure la composition de propergol solide à évaluer est proche ou éloignée d'un espace de données couvert par la base de données ayant servi à entraîner et valider le modèle d'apprentissage automatique MLMOD.
Soit $f^i$ un vecteur à $K$ dimensions pour le $i$-ème jeu de $K$ caractéristiques représentatif d'une composition de propergol solide dans la base de données. Par exemple, la dimension $K$ est 12, qui est le nombre de variables disponibles pour représenter la composition de propergol solide. Considérant un vecteur $g$ à comparer avec $f^i$, la distance de similarité $d$ du vecteur $g$ avec le vecteur $f^i$ est, dans un mode de réalisation particulier, définie par :

$$d = \sum_k w_k \cdot (f_k^i - g_k)^2$$

où $g_k$ est la valeur de la $k$-ième caractéristique ($k = 1,.., K$) du vecteur $g$, $f_k^i$ est la $k$-ième caractéristique ($k = 1,.., K$) du vecteur $f^i$, $w_k$ est un coefficient de pondération appliqué à la $k$-ième caractéristique des vecteurs comparés pour tenir compte de l'importance relative de cette $k$-ième caractéristique dans la vitesse de combustion obtenue.
Et la distance de similarité minimum $d_{min}$ du vecteur $g$ avec tout jeu de caractéristiques de propergol solide dans la base de données est alors définie par :

$$d_{min} = \min_i \sum_k w_k \cdot (f_k^i - g_k)^2$$

La distance de similarité minimum $d_{min}$ fournit ainsi une indication de la distance séparant la composition de propergol solide représentée par le vecteur $g$ et l'espace de données couvert par la base de données.
Dans un mode de réalisation particulier, les coefficients de pondération $w_k$, $k=1,..,K$, sont définis par une analyse de la base de données par un modèle d'apprentissage automatique de type forêt d'arbres décisionnels (RF, pour « Random Forest » en anglais). Ce modèle d'apprentissage automatique, nommé ici MLMOD2, est entraîné à prédire des vitesses de combustion, comme l'autre modèle d'apprentissage automatique MLMOD introduit ci-dessous. Le modèle d'apprentissage automatique MLMOD2 est entraîné sur la même base d'entrainement que le modèle d'apprentissage automatique MLMOD (et peut d'ailleurs être le même modèle). Le modèle d'apprentissage automatique MLMOD2 est avantageusement de type forêt d'arbres décisionnels alors que, comme décrit ci-après, le modèle d'apprentissage automatique MLMOD peut être d'un autre type pour plus de précision, car le type forêt d'arbres décisionnels a l'intérêt de fournir directement, de par sa structure, l'importance de chaque paramètre (la pondération) à la vitesse de combustion.
En variante, les coefficients de pondération $w_k$, $k=1,..,K$, sont définis par une analyse de la base de données par analyse en composantes principales (PCA, « Principal Component Analysis » en anglais) pour faire apparaître quelle influence ont les caractéristiques utilisées pour peupler la base de données sur la vitesse de combustion des compositions de propergol solide.
Les caractéristiques ayant le plus d'influence sur la vitesse de combustion des compositions de propergol solide bénéficient alors de coefficients de pondération augmentant leur influence sur le calcul de distance de similarité (typiquement plus élevé, mais cela dépend de la formule de distance de similarité utilisée) et celles ayant le moins d'influence sur la vitesse de combustion des compositions de propergol solide accusent des coefficients de pondération diminuant leur influence sur le calcul de distance de similarité (typiquement plus faible, mais cela dépend de la formule de distance de similarité utilisée).

Dans une étape 108, le système de traitement détermine si la distance de similarité minimum $d_{min}$ calculée à l'étape 106 est inférieure à un seuil prédéterminé TH. Si tel est le cas, le modèle d'apprentissage automatique MLMOD est utilisé et une étape 110 est effectuée ; sinon, un modèle physique est utilisé, et une étape 112 est effectuée.

Un modèle physique représente par une modélisation de lois physiques le comportement de compositions de propergols solides à la combustion. Par exemple, un modèle physique utilisable est décrit dans le document *"Physico-chemical mechanisms of solid propellant combustion."*, Lengelle, G., J. Duterque, et J. F. Trubert., Solidpropellant chemistry, combustion, and motor interior ballistics, Progress in Astronautics and Aeronautics, vol. 185 (2000), pages 287 et suivantes. Il est plus particulièrement fait référence ici aux pages 287, 305, 310, 311, 321, 323, 326, 327 et 328.

Il en ressort que les gaz dégagés par la combustion du perchlorate d'ammonium (AP) et du polybutadiène hydroxytéléchélique (HTPB) réagissent dans une flamme de diffusion qui introduit un flux de chaleur additionnel dépendant de la taille $D_{Ap,i}$ (diamètre nominal) des différentes classes de particules de perchlorate d'ammonium (AP) utilisées.

Ainsi, en s'appuyant notamment sur l'équation 56 à la page 321 de cet ouvrage, une estimation de la vitesse de combustion d'une composition de propergol solide peut être obtenue comme suit :

$$\frac{1}{r_b} = \sum_i \frac{x_{AP,i}}{r_{AP,i}(D_{AP,i})} + \frac{1 - \sum_i x_{AP,i}}{r_{HTPB,i}(D_{AP,i})}$$

où $r_b$ représente la vitesse de combustion de la composition considérée, $r_{AP,i}$ représente la vitesse de combustion pour chaque classe $i$ de taille $D_{AP,i}$ (diamètre nominal) de particules de perchlorate d'ammonium (AP), $x_{AP,i}$ la fraction volumique de particules de perchlorate d'ammonium (AP) utilisée avec la taille de classe $i$, et $r_{HTPB}$ la vitesse de combustion du polybutadiène hydroxytéléchélique (HTPB) pour chaque classe $i$ de taille $D_{AP,i}$ de particules de perchlorate d'ammonium (AP).

Si de l'aluminium est incorporé dans la composition de propergol solide, la relation ci-dessus demeure. Ce qui change réside dans le dégagement de chaleur en surface pour tenir compte de la chaleur de fusion de l'aluminium. Dans son ensemble, le modèle physique estime la vitesse de combustion $r_b$ à une pression donnée, à partir de paramètres d'entrée qui décrivent la formulation chimique de la composition : fraction d'aluminium, et pour chaque classe $i$ de taille de particules de perchlorate d'ammonium (AP), la fraction de particules $x_{AP,i}$ correspondante ainsi que leur diamètre nominal $D_{AP,i}$.

Dans un mode de réalisation particulier, le modèle d'apprentissage automatique MLMOD prend en compte plus de caractéristiques entrant dans la composition que la formulation chimique de la composition à une pression donnée, comme par exemple une indication de type de malaxeur utilisé pour créer la composition à partir de la formulation chimique.

Il est tenu compte ici de manière astucieuse que, lorsque la distance de similarité minimum $d_{min}$ est inférieure au seuil prédéterminé TH, le modèle d'apprentissage automatique MLMOD est plus performant que le modèle physique. Inversement, une valeur de distance de similarité minimum $d_{min}$ supérieure ou égale au seuil prédéterminé TH indique que la composition de propergol solide à évaluer est au-delà de l'espace des caractéristiques contenues dans la base de données utilisée pour entraîner et valider le modèle d'apprentissage automatique MLMOD, et donc qu'il existe un risque que le modèle d'apprentissage automatique MLMOD fournisse un résultat biaisé d'estimation de vitesse de combustion vis-à-vis de la composition de propergol solide à évaluer en raison d'une erreur de généralisation. Dans ce cas, le modèle physique est préféré.

En effet, les modèles d'apprentissage automatique fonctionnent généralement très bien pour interpoler les données, mais peuvent parfois échouer à extrapoler au-delà d'une plage de valeurs pour laquelle ils ont été entraînés, ce qui peut entraîner des erreurs de généralisation importantes. D'autre part, les modèles physiques sont, en moyenne, légèrement moins performants, mais sont moins sujets aux erreurs de généralisation importantes. En effet, les modèles physiques étant par définition fondés sur des modélisations de lois de la physique, il est peu probable d'obtenir des taux de combustion qui ne correspondent pas à des réalités physiques. Inversement, les modèles d'apprentissage automatique ne connaissent pas les lois de la physique et peuvent donc conduire à des résultats aberrants dans certains cas.

Ainsi, dans l'étape 110, le système de traitement fournit une estimation de la vitesse de combustion de la composition de propergol solide à évaluer, en utilisant le modèle d'apprentissage automatique MLMOD. Et dans l'étape 112, le système de traitement fournit une estimation de la vitesse de combustion de la composition de propergol solide à évaluer, en utilisant au contraire le modèle physique.

Dans un mode de réalisation particulier, le seuil TH est défini en s'appuyant sur des compositions de propergol solide dont la vitesse de combustion est connue et qui ne sont pas utilisées dans la base de données utilisée pour entraîner et valider le modèle d'apprentissage automatique MLMOD. Par exemple, des compositions académiques de propergol solide peuvent être utilisées. Notamment, lorsque la base de données se rapporte à des compositions de propergol solide plurimodales (c'est-à-dire contenant des particules oxydantes de différentes tailles), des compositions académiques monomodales de propergol solide peuvent être utilisées pour étudier des cas éloignés de l'espace de données couvert par la base de données et permettre d'ajuster le seuil TH en conséquence.

Dans un mode de réalisation particulier, le système de traitement fournit une première estimation de la vitesse de combustion de la composition de propergol solide à évaluer, en utilisant le modèle d'apprentissage automatique MLMOD, et une deuxième estimation de la vitesse de combustion de la composition de propergol solide à évaluer, en utilisant le modèle physique. Le système de traitement fournit en outre une indication indiquant quelle valeur est *a priori* plus fiable parmi la première estimation et la deuxième estimation, selon la valeur de la distance de similarité minimum $d_{min}$ par rapport au seuil TH. Le système de traitement indique alors laquelle parmi les première et deuxième estimations est privilégiée. En d'autres termes, la première estimation est mise en avant comme estimation privilégiée lorsque la distance de similarité minimum $d_{min}$ est inférieure au seuil TH et la deuxième estimation est mise en avant comme estimation privilégiée lorsque la distance de similarité minimum $d_{min}$ est supérieure ou égale au seuil TH.

Alors, lorsqu'une expérimentation est réalisée avec cette composition de propergol solide, la mesure de la vitesse de combustion du propergol solide peut être comparée avec les première et deuxième estimations, et le seuil TH est ajusté en conséquence, dans un mode de réalisation particulier, pour de prochaines évaluations de compositions de propergol solide. En d'autres termes, le seuil TH est abaissé lorsque le système de traitement a privilégié la première estimation fournie par le modèle d'apprentissage automatique MLMOD alors l'expérimentation montre que la vitesse de combustion est plus proche de la deuxième estimation fournie par le modèle physique ; et le seuil TH est augmenté lorsque le système de traitement a privilégié la deuxième estimation fournie par le modèle physique alors l'expérimentation montre que la vitesse de combustion est plus proche de la première estimation fournie par le modèle d'apprentissage automatique MLMOD.

La **Fig. 2** illustre schématiquement un exemple d'architecture matérielle d'un système SYS 200 comportant de la circuiterie électrique adaptée pour implémenter le système de traitement évoqué ici.

L'architecture matérielle comprend alors, reliés par un bus de communication 210 : un processeur ou CPU (« Central Processing Unit » en anglais) 201 ou une grappe de processeurs ; une mémoire vive RAM (« Random Access Memory » en anglais) 202 ; une mémoire morte ROM (« Read Only Memory » en anglais) 203, ou EEPROM (« Electrically Erasable Programmable ROM » en anglais), ou une mémoire de type Flash ; un support de stockage de données DSM (« Data Storage Medium » en anglais) 204, tel qu'un disque dur HDD (« Hard Disk Drive » en anglais), ou un lecteur de support de stockage, tel qu'un lecteur de cartes SD (« Secure Digital » en anglais) ; et au moins une interface I/f205, telle qu'une interface de communication ou des entrées-sorties d'interconnexion avec des périphériques de saisie et d'affichage.

Le processeur 201 est capable d'exécuter des instructions chargées dans la RAM 202 à partir de la ROM 203, d'une mémoire externe (non représentée), d'un support de stockage, tel qu'une carte SD, ou d'un réseau de communication. Lorsque l'architecture matérielle est mise sous tension, le processeur 201 est capable de lire de la RAM 202 des instructions et de les exécuter. Ces instructions forment un programme d'ordinateur causant la mise en œuvre, par le processeur 201, des étapes et algorithmes décrits ici.

Tout ou partie des étapes et algorithmes décrit ici peut ainsi être implémenté sous forme logicielle par exécution d'un ensemble d'instructions par une machine programmable, telle qu'un DSP (« Digital Signal Processor » en anglais) ou un microcontrôleur, ou être implémenté sous forme matérielle par une machine ou un composant (« chip » en anglais) ou un ensemble de composants (« chipset » en anglais), tel qu'un FPGA (« Field-Programmable Gate Array » en anglais) ou un ASIC (« Application-Specific Integrated Circuit » en anglais). D'une manière générale, le système de traitement comprend de la circuiterie électronique agencée et configurée pour implémenter les étapes et algorithmes décrits ici.

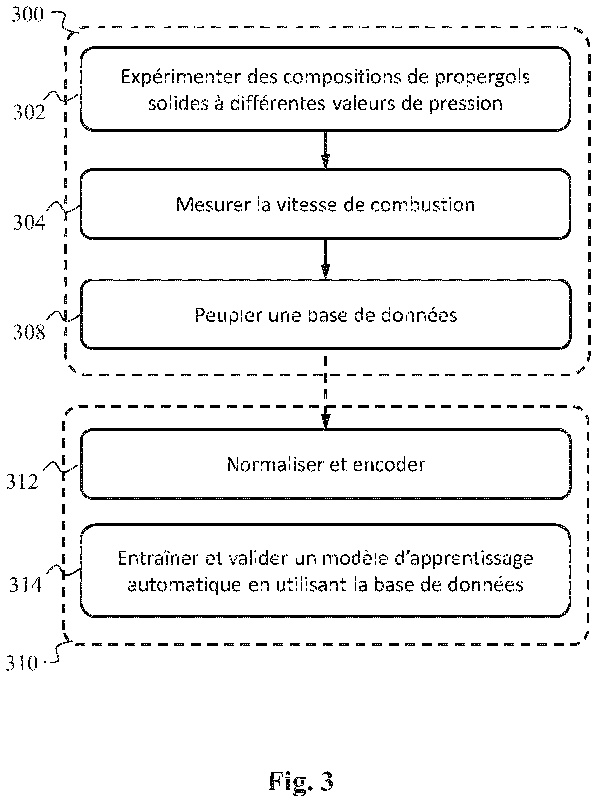

La **Fig. 3** illustre schématiquement un algorithme de mise en place de la base de données et du modèle d'apprentissage automatique, qui sont utilisés dans le procédé de la Fig. 1.

La base de données est peuplée de données collectées lors d'une phase 300 d'expérimentation, c'est-à-dire au fil d'expérimentations réalisées en laboratoire et/ou au cours d'essais.

Grâce à la constitution de cette base de données, les expérimentations ultérieures peuvent être mieux ciblées, et venir elles-mêmes enrichir encore la base de données (et donc l'apprentissage du modèle d'apprentissage automatique).

Dans une étape 302, des expérimentations sont menées avec des compositions de propergols solides à différentes valeurs de pression.

Tout ou partie des compositions de propergol solide expérimentées pour peupler la base de données a été fait l'objet d'une mesure de vitesse de combustion à différentes valeurs de pression, par exemple sur un intervalle allant de quelques hPa à plusieurs centaines de hPa (*e.g.,* 250 fois la pression atmosphérique nominale au niveau de la mer).

Dans une étape 304, la vitesse de combustion d'échantillons de propergols solides lors de ces expérimentations est mesurée.

Dans un mode de réalisation particulier, la détermination de la vitesse de combustion de chaque composition de propergol solide lors des expérimentations antérieures est réalisée de manière identique, afin d'éviter d'introduire des divergences qui seraient liées à un emploi de méthodes différentes. Préférentiellement, cette détermination de la vitesse de combustion est effectuée grâce à une technique ultrasonique, par exemple selon un principe d'impulsion-écho pour déterminer l'évolution de l'épaisseur d'un échantillon de propergol solide pendant une expérience de combustion.

Dans une étape 306, la base de données est peuplée de différentes données (*i.e.,* jeux de caractéristiques) de chaque expérimentation, y compris de la vitesse de combustion mesurée. Par exemple, la base de données est un tableur comportant, pour chaque expérimentation, les jeux de caractéristiques relatifs à l'expérimentation en question.

La base de données contient alors un ensemble de jeux de caractéristiques avec lesquels le modèle d'apprentissage automatique pour estimer des vitesses de combustion à partir de compositions de propergol solide peut être entraîné et validé. Chaque jeu de caractéristiques est ainsi représentatif d'une composition de propergol solide associée à une valeur de pression pour une vitesse de combustion correspondante, et pour lequel la vitesse de combustion est connue par expérimentation.

Dans un mode de réalisation particulier, chaque jeu de caractéristiques comporte les caractéristiques suivantes :

- la valeur de pression ;
- une indication de fraction massique de particules oxydantes pour chaque taille d'un panel de tailles possibles pour les particules oxydantes ;
- une indication de fraction massique d'aluminium potentiellement incorporé ;
- une indication de taille nominale de particules d'aluminium potentiellement incorporé ;
- une indication de nature d'un catalyseur balistique (« burning rate modifier » en anglais) potentiellement incorporé ;
- une indication de taille de particules du catalyseur balistique potentiellement incorporé.

Par exemple, le panel de tailles possibles pour des particules oxydantes de type perchlorate d'ammonium (AP) est le suivant : 400 $\mu$m, 200 $\mu$m, 90 $\mu$m, 30 $\mu$m, 10 $\mu$m, 3 $\mu$m.

Pour réaliser un échantillon / produit de propergol solide, les différentes composantes selon la composition définie sont mélangées dans un malaxeur, et bien qu'en théorie le choix du malaxeur n'intervient pas dans la vitesse de combustion du propergol solide, il se trouve qu'en pratique le choix du malaxeur peut influer la vitesse de combustion du propergol solide, notamment sa taille (ou son volume). C'est une caractéristique qui peut alors être avantageusement introduite dans la base de données. Ainsi, dans un mode de réalisation particulier, chaque jeu de caractéristiques comporte en outre la caractéristique suivante :

- une indication de type de malaxeur.

Le type de malaxeur est par exemple défini par une catégorie de taille (ou de volume de cuve) du malaxeur considéré.

Le contenu de la base de données est alors utilisé dans une phase 310 de constitution du modèle d'apprentissage automatique MLMOD susmentionné.

Dans une étape 312, optionnellement, le système de traitement normalise les différents jeux de caractéristiques de la base de données. En d'autres termes, de manière à faciliter le calcul de distance de similarité, les caractéristiques qui sont obtenues sous forme de catégories (par exemple, la nature d'un catalyseur balistique potentiellement incorporé (tel que de l'oxyde de cuivre ou de l'oxyde de fer), ou la taille du malaxeur utilisé) sont converties en valeurs numériques selon une ou plusieurs règles de conversion. Les règles de conversion respectent les dissimilarités de propriétés qui existent entre les catégories à convertir. Par exemple, un encodage de type « one-hot » peut être utilisé. L'encodage « one-hot », ou encodage 1 parmi *n,* consiste à encoder une variable à n états (catégories) sur n bits dont un seul prend une valeur égale à 1, le numéro du bit de valeur égale à 1 étant le numéro affecté à l'état (catégorie) pris par la variable. De plus, les caractéristiques qui sont déjà sous forme numérique sont ramenées à une même échelle de référence selon une ou plusieurs règles de mise à l'échelle. Par exemple, dans un intervalle de valeurs entre 0 et 10.

Dans une étape 314, le système de traitement entraîne et valide le modèle d'apprentissage automatique MLMOD grâce au contenu de la base de données (après éventuellement normalisation). Par exemple, 80% du contenu de la base de données sont utilisés pour entraîner le modèle d'apprentissage automatique MLMOD et les 20% restants sont utilisés pour valider le modèle d'apprentissage automatique MLMOD.

Dans un mode de réalisation particulier, le modèle d'apprentissage automatique MLMOD est de type réseau de neurones artificiels ANN (« Artificial Neural Network » en anglais).

Dans un autre mode de réalisation particulier, le modèle d'apprentissage automatique MLMOD est de type forêt d'arbres décisionnels extrêmes, ou ExtraTrees (« extremely randomized trees » en anglais).

Le modèle d'apprentissage automatique MLMOD peut alors être utilisé pour estimer la vitesse de combustion de compositions de propergols solides qui lui sont injectées en entrée, comme expliqué ci-dessus en relation avec la Fig. 1.

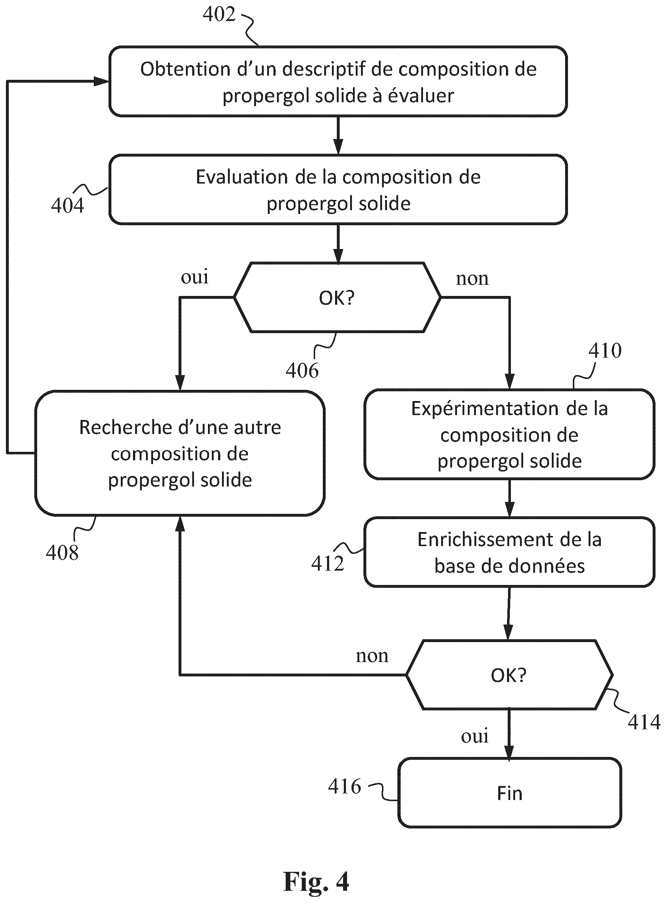

La **Fig. 4** illustre schématiquement un procédé de conduite d'expérimentations utilisant le procédé de la Fig. 1.

Dans une étape 402, un descriptif de composition de propergol solide à évaluer est obtenu.

Dans une étape 404, la composition de propergol solide est évaluée par application du procédé de la Fig. 1. Ainsi, une vitesse de combustion estimée de la composition de propergol solide à évaluer est fournie par le système de traitement.

Dans une étape 406, la vitesse de combustion estimée de la composition de propergol solide à évaluer est comparée avec une valeur cible. Si la vitesse de combustion estimée de la composition de propergol solide à évaluer est égale à la valeur cible à une marge prédéfinie près, alors une étape 410 est effectuée ; sinon, une étape 408 est effectuée.

Dans l'étape 408, une autre composition de propergol solide est recherchée, en espérant que son évaluation à l'aide du procédé de la Fig. 1 permette d'atteindre la valeur cible de vitesse de combustion souhaitée.

La recherche de cette autre composition de propergol solide peut être assistée par ordinateur. Puis, lorsqu'une telle autre composition de propergol solide est trouvée, le procédé est répété à partir de l'étape 402 avec cette autre composition de propergol solide.

Dans l'étape 410, une expérimentation de la composition de propergol solide en question est réalisée. Lors de cette expérimentation, la vitesse de combustion réelle de la composition de propergol solide en question est mesurée.

Optionnellement, dans une étape 412, il peut être décidé de conserver ou pas le jeu de caractéristiques de l'expérimentation et d'en enrichir la base de données.

Dans une étape 414, la vitesse de combustion mesurée de la composition de propergol solide expérimentée est comparée avec la valeur cible. Si la vitesse de combustion mesurée de la composition de propergol solide en question est égale à la valeur cible, alors une étape 416 est effectuée ; sinon, l'étape 408 est effectuée et une autre composition de propergol solide est recherchée.

Dans une étape 416, il est mis fin au procédé de conduite d'expérimentations, étant donné qu'une composition de propergol solide correspondant à la valeur cible de vitesse de combustion a été trouvée.

**Revendications**

**1.** Procédé pour obtenir une estimation de vitesse de combustion d'une composition de propergol solide à évaluer, le procédé étant exécuté par un système de traitement (200), le procédé comportant :

- calculer (106) une distance de similarité avec un contenu de base de données ayant servi à entraîner et valider un modèle d'apprentissage automatique pour estimer des vitesses de combustion à partir de jeux de caractéristiques représentatifs de compositions de propergol solide ;
- fournir (110) l'estimation de vitesse de combustion de la composition de propergol solide, en utilisant le modèle d'apprentissage automatique, lorsque la distance de similarité calculée est inférieure à un seuil prédéterminé ; et
- fournir (112) l'estimation de vitesse de combustion de la composition de propergol solide, en utilisant au contraire un modèle physique représentatif de lois de combustion de propergol solide, lorsque la distance de similarité calculée est supérieure ou égale au seuil prédéterminé.

**2.** Procédé selon la revendication 1, dans lequel la distance de similarité, notée $d_{min}$, est calculée de la façon suivante :

$$d_{min} = \min_i \sum_k w_k \cdot (f_k^i - g_k)^2$$

où $f_k^i$ est la valeur de la k-ième caractéristique d'un vecteur $f^i$ correspondant à un $i$-ème jeu de caractéristiques dans la base de données, $g_k$ est la valeur de la $k$-ième caractéristique d'un jeu de caractéristiques représentatif de la composition de propergol solide à évaluer $g$ et $w_k$ est un coefficient de pondération associé à la $k$-ième caractéristique.

**3.** Procédé selon la revendication 2, dans lequel les coefficients de pondération $w_k$, $k=1,..,N$, sont définis par une analyse de la base de données par un modèle d'apprentissage automatique de type forêt d'arbres décisionnels.

**4.** Procédé selon l'une quelconque des revendications 1 à 3, dans lequel le modèle d'apprentissage automatique pour estimer des vitesses de combustion à partir de compositions de propergol solide est de type réseau de neurones artificiels.

**5.** Procédé selon l'une quelconque des revendications 1 à 3, dans lequel le modèle d'apprentissage automatique pour estimer des vitesses de combustion à partir de compositions de propergol solide est de type forêt d'arbres décisionnels extrêmes.

**6.** Procédé selon l'une quelconque des revendications 1 à 5, dans lequel la base de données contient un ensemble de jeux de caractéristiques avec lesquels le modèle d'apprentissage automatique pour estimer des vitesses de combustion à partir de compositions de propergol solide a été entraîné et validé (314), chaque jeu de caractéristiques étant représentatif d'une composition de propergol solide associée à une valeur de pression pour une vitesse de combustion correspondante, chaque jeu de caractéristiques comportant :

- la valeur de pression ;
- une indication de fraction massique de particules oxydantes pour chaque taille d'un panel de tailles possibles pour les particules oxydantes ;
- une indication de fraction massique d'aluminium potentiellement incorporé ;
- une indication de taille nominale de particules d'aluminium potentiellement incorporé ;
- une indication de nature d'un catalyseur balistique potentiellement incorporé ; et
- une indication de taille de particules du catalyseur balistique potentiellement incorporé.

7. Procédé selon la revendication 6, dans lequel chaque jeu de caractéristiques comporte en outre :

- une indication de type de malaxeur utilisé.

8. Procédé selon la revendication 7, dans lequel l'indication de type de malaxeur utilisé est une indication de catégorie de taille de malaxeur utilisé.

9. Procédé selon l'une quelconque des revendications 1 à 8, dans lequel une première estimation de vitesse de combustion de la composition de propergol solide est fournie en utilisant le modèle d'apprentissage automatique et une deuxième estimation de vitesse de combustion de la composition de propergol solide est fournie en utilisant le modèle physique, et dans lequel ladite première estimation est mise en avant comme estimation privilégiée lorsque la distance de similarité calculée est inférieure au seuil prédéterminé et ladite deuxième estimation est mise en avant comme estimation privilégiée lorsque la distance de similarité calculée est supérieure ou égale au seuil prédéterminé.

10. Procédé d'expérimentation pour rechercher une composition de propergol solide qui respecte une vitesse de combustion spécifiée, comportant :

- obtenir (402) un descriptif d'une composition de propergol solide à évaluer ;
- obtenir (404) une estimation de vitesse de combustion de la composition de propergol solide à évaluer en appliquant le procédé selon l'une quelconque des revendications 1 à 9 ;
- lorsque l'estimation de vitesse de combustion de la composition de propergol solide à évaluer respecte une vitesse de combustion spécifiée avec une marge prédéterminée, expérimenter (410) la composition de propergol solide en question, sinon rechercher (408) une autre composition de propergol solide à évaluer, et réitérer.

11. Produit programme d'ordinateur comportant des instructions pour implémenter le procédé selon l'une quelconque des revendications 1 à 9, lorsque les instructions sont exécutées par un processeur.

12. Support de stockage d'informations stockant des instructions pour implémenter le procédé selon l'une quelconque des revendications 1 à 9, lorsque les instructions sont exécutées par un processeur.

13. Système de traitement (200) comportant de la circuiterie électronique configurée pour obtenir une estimation de vitesse de combustion d'une composition de propergol solide à évaluer, en :

- calculant (106) une distance de similarité avec un contenu de base de données ayant servi à entraîner et valider un modèle d'apprentissage automatique pour estimer des vitesses de combustion à partir de compositions de propergol solide ;
- fournissant (110) l'estimation de vitesse de combustion de la composition de propergol solide, obtenue en utilisant le modèle d'apprentissage automatique, lorsque la distance de similarité calculée est inférieure à un seuil prédéterminé ; et
- fournissant (112) l'estimation de vitesse de combustion de la composition de propergol solide, obtenue en utilisant un modèle physique représentatif de lois de combustion de propergol solide, lorsque la distance de similarité calculée est supérieure ou égale à un seuil prédéterminé.

102 ⌇ Entrée d'un descriptif d'une composition de propergol solide à évaluer

104 ⌇ Conversion sous forme de vecteur

106 ⌇ Calcul d'une distance de similarité

oui

Distance de similarité inférieure à un seuil prédéterminé ?

non

110

108

112

Estimation de vitesse de combustion par un modèle d'apprentissage automatique

Estimation de vitesse de combustion par un modèle physique

**Fig. 1**

**201**
CPU

**202**
RAM

**203**
ROM

**200**
SYS

**204**
DSM

**205**
I/f

210

**Fig. 2**

300

302 — Expérimenter des compositions de propergols solides à différentes valeurs de pression

304 — Mesurer la vitesse de combustion

308 — Peupler une base de données

312 — Normaliser et encoder

314 — Entraîner et valider un modèle d'apprentissage automatique en utilisant la base de données

310

**Fig. 3**

```
                          402
        ┌──────────────────────────────────────────┐
        │   Obtention d'un descriptif de composition de │
        │         propergol solide à évaluer           │
        └──────────────────────────────────────────┘
                              │
                              ▼
        ┌──────────────────────────────────────────┐
   404  │   Evaluation de la composition de            │
        │         propergol solide                     │
        └──────────────────────────────────────────┘
```

Obtention d'un descriptif de composition de propergol solide à évaluer — 402

Evaluation de la composition de propergol solide — 404

oui ← OK? → non — 406

Recherche d'une autre composition de propergol solide — 408

Expérimentation de la composition de propergol solide — 410

Enrichissement de la base de données — 412

non ← OK? — 414

oui

Fin — 416

**Fig. 4**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LENGELLE, G** ; **J. DUTERQUE** ; **J. F. TRUBERT.** Physico-chemical mechanisms of solid propellant combustion.. *Solidpropellant chemistry, combustion, and motor interior ballistics, Progress in Astronautics and Aeronautics*, 2000, vol. 185, 287